(19) Europäisches Patentamt / European Patent Office / Office européen des brevets

(11) **EP 2 556 751 B1**

(12) **EUROPEAN PATENT SPECIFICATION**

(45) Date of publication and mention
of the grant of the patent:
**24.02.2016 Bulletin 2016/08**

(21) Application number: **11765975.5**

(22) Date of filing: **07.04.2011**

(51) Int Cl.:
***A23D 9/007*** *(2006.01)*

(86) International application number:
**PCT/JP2011/058817**

(87) International publication number:
**WO 2011/126075 (13.10.2011 Gazette 2011/41)**

(54) **FAT OR OIL COMPOSITION**

FETT- ODER ÖLZUSAMMENSETZUNG

COMPOSITION D'HUILE OU DE MATIÈRE GRASSE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.05.2010   JP 2010113906
08.04.2010   JP 2010089645**

(43) Date of publication of application:
**13.02.2013   Bulletin 2013/07**

(73) Proprietor: **Kao Corporation
Chuo-Ku
Tokyo 103-8210 (JP)**

(72) Inventor: **SHIMIZU, Masao
Tokyo 131-8501 (JP)**

(74) Representative: **Hoffmann Eitle
Patent- und Rechtsanwälte PartmbB
Arabellastraße 30
81925 München (DE)**

(56) References cited:
**EP-A1- 1 262 173        JP-A- 2001 354 556
JP-A- 2004 359 784     JP-A- 2005 261 357**

**Description**

Field of the Invention

The present invention relates to a vitaminB1-containing oil or fat composition and a nicotinamide-containing oil or fat composition.

Background of the Invention

An oil or fat is one of the important nutrients which include protein and carbohydrate, and is particularly useful as an energy source. The oil or fat is also used as a heating medium or the like in cooking. The oil or fat is also an important material imparting its good taste and flavor to food. With an increase in health consciousness in recent years, attempts have been made to increase appeal power of merchandise by adding nutrient components to an edible oil. For example, there is reported a stabilized tocopherol-containing oil or fat composition containing $\alpha$-tocopherol and $\beta$-tocopherol each having a high physiological activity at a high content (Patent Document 1).

The tocopherol is one of the vitamin Es, which are lipid- soluble vitamins, and is a component that is intrinsically contained in oil or fat as a natural component. However, many nutrient components are not soluble in oil and are not added in an amount originally intended in many cases.

On the other hand, vitamin B1 is one of the water-soluble vitamins which are involved in carbohydrate metabolism, normalization of neuronal function, or the like. It is important for humans to take a suitable amount of vitamin B1 for maintaining their health. However, an amount of vitamin B1 to be ingested tends to be insufficient because of an increase in rate of meal skipping, dependence on processed food, or the like.

Further, nicotinamide is one of the water-soluble vitamins collectively called niacin (vitamin B3), which also include nicotinic acid. Nicotinamide is involved in carbohydrate metabolism, normalization of neuronal function, or the like as a coenzyme working in a metabolic system of carbohydrate/lipid/protein in a form of nicotinamide adenine dinucleotide (NAD) or nicotinamide adenine dinucleotide phosphate (NADP). It is important for humans to ingest a suitable amount of nicotinamide for maintaining their health. However, an amount of nicotinamide to be ingested tends to be insufficient because of an increase in rate of meal skipping, dependence on processed food, or the like.

When vitamin B1 and nicotinamide are supplied from nutrient-enriched food, it is desirable to be able to ingest vitamin B1 and nicotinamide each in an amount equal to or more than one third of the recommended dietary allowance per day on the basis of "Recommended Dietary Allowances for the Japanese, Sixth Revision-Dietary References Intakes."

Prior Art Document

Patent Document

[Patent Document 1] JP-A-8-173035

Summary of the Invention

The present invention relates to the following items 1) to 60).

1) An oil or fat composition, including a vitamin B1 derivative or a salt thereof at a content of 44 to 8, 000 ppm in terms of thiamine and having a hydroxyl value of 9 to 100 mg-KOH/g,

in which the content C (ppm) of the vitamin B1 derivative or the salt thereof in terms of thiamine and the hydroxyl value X (mg-KOH/g) satisfy a relationship of the following expression (1) .

$$[Ln(C/143)]/X \leq 0.044 \ (Ln: \ natural \ logarithm) \qquad (1)$$

2) The oil or fat composition according to the above-mentioned item 1),

in which the content C (ppm) of the vitamin B1 derivative or the salt thereof in terms of thiamine and the hydroxyl value X (mg-KOH/g) satisfy a relationship of the following expression (2) .

$$[Ln(C/82)]/X \leq 0.04 \quad (Ln: \text{ natural logarithm}) \qquad (2)$$

3) The oil or fat composition according to the above-mentioned item 1) or 2), in which the content of the vitamin B1 derivative or the salt thereof is 44 to 5,000 ppm in terms of thiamine.

4) The oil or fat composition according to the above-mentioned item 1) or 2), in which the content of the vitamin B1 derivative or the salt thereof is 44 to 3,000 ppm in terms of thiamine.

5) The oil or fat composition according to the above-mentioned item 1) or 2), in which the content of the vitamin B1 derivative or the salt thereof is 150 to 3,000 ppm in terms of thiamine.

6) The oil or fat composition according to any one of the above-mentioned items 1) to 5), which has a hydroxyl value of 20 to 100 mg-KOH/g.

7) The oil or fat composition according to any one of the above-mentioned items 1) to 5), which has a hydroxyl value of 40 to 100 mg-KOH/g.

8) The oil or fat composition according to the above-mentioned item 1) or 2), in which the content of the vitamin B1 derivative or the salt thereof is 500 to 8,000 ppm in terms of thiamine and which has a hydroxyl value of 30 to 100 mg-KOH/g.

9) The oil or fat composition according to the above-mentioned item 1) or 2), in which the content of the vitamin B1 derivative or the salt thereof is 500 to 8,000 ppm in terms of thiamine and which has a hydroxyl value of 50 to 100 mg-KOH/g.

10) The oil or fat composition according to the above-mentioned item 1) or 2), in which the content of the vitamin B1 derivative or the salt thereof is 1,000 to 8,000 ppm in terms of thiamine and which has a hydroxyl value of 50 to 100 mg-KOH/g.

11) The oil or fat composition according to the above-mentioned item 1) or 2), in which the content of the vitamin B1 derivative or the salt thereof is 1,000 to 8,000 ppm in terms of thiamine and which has a hydroxyl value of 66 to 100 mg-KOH/g.

12) The oil or fat composition according to the above-mentioned item 1) or 2), in which the content of the vitamin B1 derivative or the salt thereof is 1,500 to 8,000 ppm in terms of thiamine and which has a hydroxyl value of 60 to 100 mg-KOH/g.

13) The oil or fat composition according to the above-mentioned item 1) or 2), in which the content of the vitamin B1 derivative or the salt thereof is 1,500 to 8,000 ppm in terms of thiamine and which has a hydroxyl value of 76 to 100 mg-KOH/g.

14) The oil or fat composition according to any one of the above-mentioned items 1) to 13), in which the vitamin B1 derivative includes bisbentiamine disulfide.

15) The oil or fat composition according to any one of the above-mentioned items 1) to 14), including diacylglycerols at 10 to 99 mass%.

16) The oil or fat composition according to any one of the above-mentioned items 1) to 14), including diacylglycerols at 20 to 90 mass%.

17) The oil or fat composition according to any one of the above-mentioned items 1) to 14), including diacylglycerols at 40 to 85 mass%.

18) The oil or fat composition according to any one of the above-mentioned items 1) to 17), further including vitamin C, a derivative thereof, or nicotinamide.

19) The oil or fat composition according to the above-mentioned item 18), in which the content of the vitamin C or the derivative thereof is 420 to 8,400 ppm in terms of ascorbic acid.

20) The oil or fat composition according to the above-mentioned item 18), in which the content of the vitamin C or the derivative thereof is 2,100 to 8,400 ppm in terms of ascorbic acid.

21) The oil or fat composition according to the above-mentioned item 18), in which the content of nicotinamide is 1,000 to 20,000 ppm.

22) The oil or fat composition according to the above-mentioned item 18), in which the content of nicotinamide is 1,000 to 6,000 ppm.

23) An oil or fat composition, including nicotinamide at a content of 1,000 to 20,000 ppm and having a hydroxyl value of 9 to 100 mg-KOH/g,

in which the content C (ppm) of nicotinamide and the hydroxyl value X (mg-KOH/g) satisfy a relationship of the following expression (3).

$$[Ln(C/850)]/X \leq 0.038 \quad (Ln: \text{ natural logarithm}) \qquad (3)$$

24) The oil or fat composition according to the above-mentioned item 23),

in which the content C (ppm) of nicotinamide and the hydroxyl value X (mg-KOH/g) satisfy a relationship of the following expression (4).

$$[Ln(C/679)]/X \leq 0.039 \text{ (Ln: natural logarithm)} \qquad (4)$$

25) The oil or fat composition according to the above-mentioned item 23),

in which the content C (ppm) of nicotinamide and the hydroxyl value X (mg-KOH/g) satisfy a relationship of the following expression (5).

$$[Ln(C/854)]/X \leq 0.031 \text{ (Ln: natural logarithm)} \qquad (5)$$

26) The oil or fat composition according to the above-mentioned item 23),

in which the content C (ppm) of nicotinamide and the hydroxyl value X (mg-KOH/g) satisfy a relationship of the following expression (6).

$$[Ln(C/859)]/X \leq 0.024 \text{ (Ln: natural logarithm)} \qquad (6)$$

27) The oil or fat composition according to any one of the above-mentioned items 23) to 26), in which the content of nicotinamide is 1,000 to 15,000 ppm.
28) The oil or fat composition according to any one of the above-mentioned items 23) to 26), in which the content of nicotinamide is 1,000 to 6,000 ppm.
29) The oil or fat composition according to any one of the above-mentioned items 23) to 26), inwhich the content of nicotinamide is 1,000 to 4,500 ppm.
30) The oil or fat composition according to any one of the above-mentioned items 23) to 29), which has a hydroxyl value of 20 to 100 mg-KOH/g.
31) The oil or fat composition according to any one of the above-mentioned items 23) to 29), which has a hydroxyl value of 40 to 100 mg-KOH/g.
32) The oil or fat composition according to any one of the above-mentioned items 23) to 26), in which the content of nicotinamide is 2,000 to 20,000 ppm and which has a hydroxyl value of 28 to 100 mg-KOH/g.
33) The oil or fat composition according to any one of the above-mentioned items 23) to 26), in which the content of nicotinamide is 2,000 to 20,000 ppm and which has a hydroxyl value of 35 to 100 mg-KOH/g.
34) The oil or fat composition according to any one of the above-mentioned items 23) to 26), in which the content of nicotinamide is 5,000 to 20,000 ppm and which has a hydroxyl value of 51 to 100 mg-KOH/g.
35) The oil or fat composition according to any one of the above-mentioned items 23) to 26), in which the content of nicotinamide is 5,000 to 20,000 ppm and which has a hydroxyl value of 73 to 100 mg-KOH/g.
36) The oil or fat composition according to any one of the above-mentioned items 23) to 26), in which the content of nicotinamide is 6,000 to 20,000 ppm and which has a hydroxyl value of 55 to 100 mg-KOH/g.
37) The oil or fat composition according to any one of the above-mentioned items 23) to 26), in which the content of nicotinamide is 6,000 to 20,000 ppm and which has a hydroxyl value of 80 to 100 mg-KOH/g.
38) The oil or fat composition according to any one of the above-mentioned items 23) to 26), inwhich the content of nicotinamide is 2,000 to 20,000 ppm and which has a hydroxyl value of 22 to 100 mg-KOH/g.
39) The oil or fat composition according to any one of the above-mentioned items 23) to 26), in which the content of nicotinamide is 2,000 to 20,000 ppm and which has a hydroxyl value of 27 to 100 mg-KOH/g.
40) The oil or fat composition according to any one of the above-mentioned items 23) to 26), in which the content of nicotinamide is 5,000 to 20,000 ppm and which has a hydroxyl value of 46 to 100 mg-KOH/g.
41) The oil or fat composition according to any one of the above-mentioned items 23) to 26), in which the content of nicotinamide is 6,000 to 20,000 ppm and which has a hydroxyl value of 51 to 100 mg-KOH/g.
42) The oil or fat composition according to any one of the above-mentioned items 23) to 41), including diacylglycerols at 10 to 99 mass%.
43) The oil or fat composition according to any one of the above-mentioned items 23) to 41), including diacylglycerols at 20 to 90 mass%.

44) The oil or fat composition according to any one of the above-mentioned items 23) to 41), including diacylglycerols at 40 to 85 mass%.

45) The oil or fat composition according to any one of the above-mentioned items 23) to 44), further including a vitamin B1 derivative or a salt thereof.

46) The oil or fat composition according to the above-mentioned item 45), in which the content of the vitamin B1 derivative or the salt thereof is 44 ppm or more in terms of thiamine.

47) The oil or fat composition according to the above-mentioned item 45), in which the content of the vitamin B1 derivative or the salt thereof is 44 to 5,000 ppm in terms of thiamine.

48) The oil or fat composition according to the above-mentioned item 45), in which the content of the vitamin B1 derivative or the salt thereof is 44 to 3,000 ppm in terms of thiamine.

49) The oil or fat composition according to the above-mentioned item 45), in which the content of the vitamin B1 derivative or the salt thereof is 44 to 2,000 ppm in terms of thiamine.

50) The oil or fat composition according to any one of the above-mentioned items 45) to 49), in which the vitamin B1 derivative includes bisbentiamine disulfide.

51) The oil or fat composition according to any one of the above-mentioned items 1) to 50), which is an edible oil.

52) A method for suppressing a bitterness of a vitamin B1 derivative, a salt thereof, or nicotinamide, which includes adding the vitamin B1 derivative, the salt thereof, or nicotinamide to an oil or fat composition having a hydroxyl value of 9 to 100 mg-KOH/g.

53) The method according to the above-mentioned item 52), in which the oil or fat composition has a hydroxyl value of 20 to 100 mg-KOH/g.

54) The method according to the above-mentioned item 52), in which the oil or fat composition has a hydroxyl value of 40 to 100 mg-KOH/g.

55) The method according to any one of the above-mentioned items 52) to 54), in which the oil or fat composition includes diacylglycerols at 10 to 99 mass%.

56) The method according to any one of the above-mentioned items 52) to 54), in which the oil or fat composition includes diacylglycerols at 20 to 90 mass%.

57) The method according to any one of the above-mentioned items 52) to 54), in which the oil or fat composition includes diacylglycerols at 40 to 85 mass%.

58) Use of an oil or fat composition having a hydroxyl value of 9 to 100 mg-KOH/g for suppressing a bitterness of a vitamin B1 derivative, a salt thereof, or nicotinamide.

59) The use according to the above-mentioned item 58), in which the oil or fat composition has a hydroxyl value of 20 to 100 mg-KOH/g.

60) The use according to the above-mentioned item 58), in which the oil or fat composition has a hydroxyl value of 40 to 100 mg-KOH/g. Embodiment for Carrying out the Invention

Vitamin B1 has low solubility and takes on an inherent bitterness, and hence its blending amount may have to be restricted. Further, vitamin B1 has a problem in that blending vitamin B1 in food or the like leads to the deterioration of the taste and flavor thereof. The inventor of the present invention has tried to dissolve a vitamin B1 derivative in an edible oil which has been conventionally used and includes triacylglycerols as main constituent components, but has found that a sufficient amount of the vitamin B1 derivative cannot be dissolved therein.

Thus, the present invention relates to providing an oil or fat composition which has a less bitterness and dissolves vitamin B1 therein at a high concentration.

On the other hand, the inventor of the present invention has tried to dissolve nicotinamide in an edible oil which has been conventionally used and includes triacylglycerols as main constituent components, but has found that an intended amount of nicotinamide cannot be dissolved therein.

Therefore, the present invention relates to providing an oil or fat composition which dissolves nicotinamide therein at a high concentration.

The inventor of the present invention has found that an oil or fat composition having a hydroxyl value (OHV) at a certain level or higher, in which the content of a vitamin B1 derivative and the hydroxyl value satisfy a predetermined relationship, can dissolve the vitamin B1 derivative therein at a high concentration, and has also found that the oil or fat composition has a less bitterness even though it includes the vitamin B1 derivative at a high concentration.

Further, the inventor of the present invention has made intensive studies. As a result, the inventor has found that an oil or fat composition having a hydroxyl value (OHV) at a certain level or higher, in which the content of nicotinamide and the hydroxyl value satisfy a predetermined relationship, can dissolve the nicotinamide therein at a high concentration.

According to the present invention, it is possible to provide an oil or fat composition which has a less bitterness and includes vitamin B1 dissolved at a high concentration, as well as an oil or fat composition which includes nicotinamide dissolved at a high concentration.

The oil or fat composition according to the present invention has a hydroxyl value (OHV) of preferably 9 to 100 mg-KOH/g, more preferably 20 to 100 mg-KOH/g, even more preferably 40 to 100 mg-KOH/g, from the viewpoints of improving the solubility of vitamin B1 and nicotinamide and of suppressing the bitterness of vitamin B1.

Herein, the hydroxyl value refers to a value measured by measurement in accordance with "Hydroxyl value (pyridine-acetic anhydride method 2.3.6.2-1996)" in "Standard Methods for the Analysis of Fats, Oils and Related Materials, 2003" edited by Japan Oil Chemists' Society.

The details of the measurement method for the hydroxyl value are described in Examples.

The oil or fat composition according to the present invention having a hydroxyl value (OHV) of 9 to 100 mg-KOH/g may be prepared by using an oil or fat, an emulsifier or the like alone, or in appropriate combination, so that the oil or fat composition has a hydroxyl value in the range described above. It is preferred to use an oil or fat highly containing monoacylglycerols and/or diacylglycerols.

Note that in the present invention, the term "oil or fat" refers to an oil or fat containing one or more of glycerols among triacylglycerols, diacylglycerols, and monoacylglycerols.

The content of diacylglycerols in the oil or fat composition according to the present invention is preferably 10 mass% (hereinafter, simply referred to as "%") or more, more preferably 20% or more, even more preferably 40% or more, from the viewpoint of improving the solubility of vitamin B1 and nicotinamide. The upper limit of the content of diacylglycerols is not particularly defined, and is preferably 99% or less, more preferably 98% or less, even more preferably 97% or less. The content of diacylglycerols in the oil or fat composition according to the present invention is preferably 10 to 99%, more preferably 20 to 90%, even more preferably 40 to 85%.

Further, the content of monoacylglycerols in the oil or fat composition according to the present invention is preferably 0 to 5%, more preferably 0 to 2%, even more preferably 0.1 to 2%, from the viewpoint of improving the solubility of vitamin B1 and nicotinamide, the viewpoint of its taste and flavor as an edible oil, and the viewpoint of its industrial productivity.

An oil or fat in the oil or fat composition according to the present invention may be produced from any of vegetable oils or fats and animal oils or fats as a raw material. Specific examples of the raw material include vegetable oils or fats such as soybean oil, rapeseed oil, safflower oil, rice bran oil, corn oil, palm oil, sunflower oil, cottonseedoil, olive oil, sesame oil, orperilla oil, animal oils or fats such as fish oils, lard, beef tallow, or butter fat, and oils or fats such as transesterified oils, hydrogenated oils, or fractionated oils thereof. In particular, unhydrogenated oils or fats are preferred from the viewpoint of reducing the content of trans-unsaturated fatty acids in all constituent fatty acids of edible oils or fats.

The constituent fatty acids of an oil or fat in the oil or fat composition according to the present invention are not particularly limited, and any of saturated fatty acids and unsaturated fatty acids may be used. Unsaturated fatty acids account for preferably 40 to 100%, more preferably 80 to 100%, even more preferably 90 to 100% of the constituent fatty acids, from the viewpoints of the outer appearance of the resulting oil or fat composition and the industrial productivity of the oil or fat. The number of carbons in the unsaturated fatty acids is preferably 14 to 24 , more preferably 16 to 22 from the viewpoint of the physiological effects.

Further, in the constituent fatty acids of an oil or fat in the oil or fat composition, the content of saturated fatty acids is preferably less than 60%, more preferably 0 to 20%, even more preferably 0 to 10%, from the viewpoints of its outer appearance, its physiological effects, and the industrial productivity of the oil or fat. The saturated fatty acids have preferably 14 to 24 carbon atoms, more preferably 16 to 22 carbon atoms.

In the constituent fatty acids of an oil or fat in the oil or fat composition, the content of trans-unsaturated fatty acids is 0 to 4%, preferably 0.1 to 3.5%, more preferably 0.2 to 3%, from the viewpoints of its taste and flavor, its physiological effects, its outer appearance, and the industrial productivity of the oil or fat.

As examples of an emulsifier used in the oil or fat composition of the present invention, there are given, in addition to monoacylglycerols and diacylglycerols, polyol fatty acid esters such as polyglycerin condensed licinoleic acid esters, polyglycerin fatty acid esters, sucrose fatty acid esters, sorbitan fatty acid esters, polyoxyethylene sorbitan fatty acid esters, or propylene glycol fatty acid esters.

(Vitamin B1-containing oil or fat composition)

The oil or fat composition containing vitamin B1 dissolved at a high concentration according to the present invention preferably includes a vitamin B1 derivative or a salt thereof at a content of 44 to 8,000 ppm in terms of thiamine, and the content thereof is more preferably 44 to 5,000 ppm, more preferably 44 to 3,000 ppm, even more preferably 150 to 3,000 ppm, from the viewpoints of its stability and physiological effects. The content of the vitamin B1 derivative in the oil or fat composition may be measured in accordance with the method described in Examples.

Examples of the vitamin B1 derivative or the salt thereof in the present invention include bisbentiamine disulfide, benfotiamine, fursultiamine, octothiamine, dibenzoyl thiamine, cycotiamine, sulbutiamine, acetiamine, dicethiamine, or salts thereof. The salts may be a pharmaceutically acceptable salt. Examples thereof include: mineral acid salts such as a nitrate, a hydrochloride, or a sulfate; or organic acid salts such as an acetate, a propionate, a tartrate, a fumarate, amaleate, amalate, a citrate, a methanesulfonate, a p-toluenesulfonate, or a trifluoroacetate. One kind or two or more kinds thereof may be appropriately selected and used. Of those, it is preferred to use a vitamin B1 derivative, in particular bisbentiamine disulfide, from the viewpoints of its solubility and physiological effects.

The vitamin B1 derivatives or the salts thereof are known compounds, and any of commercially available compounds may be used or it is possible to produce a vitamin B1 derivative or a salt thereof on the basis of a known method.

In the oil or fat composition containing vitamin B1 dissolved at a high concentration according to the present invention, the content C (ppm) of a vitamin B1 derivative or a salt thereof in terms of thiamine and the hydroxyl value X (mg-KOH/g) satisfy a relationship of the following expression (1).

$$[Ln(C/143)]/X \leq 0.044 \ (Ln:\ natural\ logarithm) \qquad (1)$$

Ln: natural logarithm

Further, the inventor of the present invention has found that when, in the oil or fat composition according to the present invention, the content C (ppm) of a vitamin B1 derivative or a salt thereof in terms of thiamine and the hydroxyl value X (mg-KOH/g) satisfy the following expression (2), the bitterness derived from the vitamin B1 derivative can be further suppressed.

$$[Ln(C/82)]/X \leq 0.04 \qquad (2)$$

Ln: the same as described above

That is, in the present invention, the amount of a vitamin B1 derivative to be dissolved in an oil or fat composition depends on the hydroxyl value thereof. Specifically, when the content C of a vitamin B1 derivative or a salt thereof in terms of thiamine is 44 ppm or more and 8,000 ppm or less, the hydroxyl value of the oil or fat composition is 9 mg-KOH/g or more and 100 mg-KOH/g or less. Meanwhile, when the content C is 500 ppm or more and 8,000 ppm or less, the hydroxyl value is preferably 30 mg-KOH/g or more, more preferably 50 mg-KOH/g or more, from the viewpoints of improving the solubility of vitamin B1 and suppressing its bitterness.

Further, when the content C is 1,000 ppm or more and 8,000 ppm or less, the hydroxyl value is preferably 50 mg-KOH/g or more, more preferably 66 mg-KOH/g or more, from the same viewpoints. When the content C is 1,500 ppm or more and 8,000 ppm or less, the hydroxyl value is preferably 60 mg-KOH/g or more, more preferably 76 mg-KOH/g or more, from the same viewpoints.

The oil or fat composition containing vitamin B1 dissolved at a high concentration according to the present invention may further contain vitamin C, a derivative thereof, or nicotinamide dissolved therein. Examples of the vitamin C or the derivative thereof include ascorbic acid, ascorbyl palmitate, or ascorbyl stearate. Nicotinamide represents one kind of niacin belonging to the vitamin B group.

The content of vitamin C or a derivative thereof in the oil or fat composition is preferably 420 to 8,400 ppm, more preferably 2,100 to 8,400 ppm in terms of ascorbic acid. Further, the content of nicotinamide is preferably 1,000 to 20,000 ppm, more preferably 1,000 to 6,000 ppm.

(Nicotinamide-containing oil or fat composition)

The oil or fat composition containing nicotinamide dissolved at a high concentration according to the present invention preferably includes nicotinamide at a content of 1,000 to 20,000 ppm, and the content thereof is more preferably 1,000 to 15,000 ppm, more preferably 1,000 to 6,000 ppm, even more preferably 1,000 to 4,500 ppm, from the viewpoints of its stability and physiological effects. The content of nicotinamide in the oil or fat composition may be measured in accordance with the method described in Examples.

A commercially available product of nicotinamide may be used in the present invention.

In the oil or fat composition according to the present invention, the content C (ppm) of nicotinamide and the hydroxyl value X (mg-KOH/g) satisfy a relationship of the following expression (3).

$$[Ln(C/850)]/X \leq 0.038 \qquad (3)$$

Ln: natural logarithm

Further, when, in the oil or fat composition according to the present invention, the content C (ppm) of nicotinamide and the hydroxyl value X (mg-KOH/g) satisfy a relationship of the following expression (4), the amount of the nicotinamide to be dissolved in the oil or fat composition can be further increased.

$$[Ln(C/679)]/X \leq 0.039 \qquad (4)$$

C: the same as described above
Ln: the same as described above

Further, the inventor of the present invention has found that when the content C (ppm) of nicotinamide and the hydroxyl value X (mg-KOH/g) satisfy the following expression (5), not only the amount of the nicotinamide to be dissolved in the oil or fat composition can be increased, but also the bitterness derived from nicotinamide can be suppressed.

$$[Ln(C/854)]/X \leq 0.031 \qquad (5)$$

C: the same as described above
Ln: the same as described above

Further, the inventor of the present invention has found that when the content C (ppm) of nicotinamide and the hydroxyl value X (mg-KOH/g) satisfy the following expression (6), the bitterness derived from nicotinamide can be further suppressed.

$$[Ln(C/859)]/X \leq 0.024 \qquad (6)$$

C: the same as described above
Ln: the same as described above

That is, in the present invention, the amount of nicotinamide to be dissolved in an oil or fat composition depends on the hydroxyl value thereof. Specifically, when the content of nicotinamide is 1,000 ppm or more and 20, 000 ppm or less, the hydroxyl value of the oil or fat composition is 9 mg-KOH/g or more and 100 mg-KOH/g or less. Meanwhile, when the content of nicotinamide is 2,000 ppm or more and 20,000 ppm or less, the hydroxyl value is preferably 28 mg-KOH/g or more, more preferably 35 mg-KOH/g or more, from the viewpoints of improving the solubility of nicotinamide and suppressing its bitterness.

Further, when the content of nicotinamide is 5,000 ppm or more and 20, 000 ppm or less, the hydroxyl value is preferably 51 mg-KOH/g or more, more preferably 73 mg-KOH/g or more, from the same viewpoints. When the content of nicotinamide is 6,000 ppm or more and 20,000 ppm or less, the hydroxyl value is preferably 55 mg-KOH/g or more, more preferably 80 mg-KOH/g or more, from the same viewpoints.

Further, in the present invention, when the content of nicotinamide is 2, 000 ppm or more and 20, 000 ppm or less, the hydroxyl value is preferably 22 mg-KOH/g or more, more preferably 27 mg-KOH/g or more, from the viewpoints of improving the solubility of nicotinamide and suppressing its bitterness.

Further, when the content of nicotinamide is 5, 000 ppm or more and 20,000 ppm or less, the hydroxyl value is preferably 46 mg-KOH/g or more, from the viewpoint of improving the solubility of nicotinamide. When the content of nicotinamide is 6, 000 ppm or more and 20, 000 ppm or less, the hydroxyl value is preferably 51 mg-KOH/g or more, from the same viewpoint.

The oil or fat composition containing nicotinamide dissolved at a high concentration according to the present invention may further include a vitamin B1 derivative or a salt thereof. Vitamin B1 is poorly dissolved in an oil or fat containing triacylglycerols as main constituent components, like nicotinamide. Examples of the vitamin B1 derivative or the salt thereof include bisbentiamine disulfide, benfotiamine, fursultiamine, octothiamine, dibenzoyl thiamine, cycotiamine, sulbutiamine, acetiamine, dicethiamine, or the salts thereof. The salts may be a pharmaceutically acceptable salt. Examples thereof include: mineral acid salts such as a nitrate, a hydrochloride, or a sulfate; and organic acid salts such as an acetate, a propionate, a tartrate, a fumarate, a maleate, a malate, a citrate, a methanesulfonate, a p-toluenesulfonate, or a trifluoroacetate. Of those, it is preferred to use a vitamin B1 derivative, in particular, bisbentiamine disulfide, from the viewpoints of its solubility and physiological effects.

The content of a vitamin B1 derivative or a salt thereof is preferably 44 ppm or more, more preferably 44 to 5,000 ppm, more preferably 44 to 3,000 ppm, even more preferably 44 to 2,000 ppm in terms of thiamine, from the viewpoint of the physiological effects thereof.

An antioxidant may be further added to the oil or fat composition according to the present invention as is the case with a common edible oil or fat, from the viewpoint of improving its storage stability and the stability of its taste and flavor. Examples of the antioxidant include natural antioxidants, tocopherol, BHT, BHA, phospholipids, organic carboxylic acids, or polyphenols or the like.

The amount of water in the oil or fat composition according to the present invention is preferably 15, 000 ppm or less, more preferably 10,000 ppm or less, even more preferably 3,000 ppm or less, from the viewpoints of its storage stability and its usability at the time of cooking.

The oil or fat composition according to the present invention may be used in the same applications as a common edible oil or fat, and may be applied to various foods and drinks, foodstuffs, and drugs. Examples of the foods and drinks include: oil-in-water type oil or fat processed foods such as drink, dessert, ice cream, dressing, toppings, mayonnaise, or sauce for grilled meat; water-in-oil type oil or fat processed foods such as margarine or spread; processed oil or fat foods such as peanut butter, frying shortening, or baking shortening; processed foods such as potato chips, snacks, cake, cookies, pie, bread, or chocolate; bakery mixes; processed meat products; frozen entrees; or frozen foods or the like.

Examples

(Analysis method)

(i) Glyceride composition

About 10 mg of an oil or fat sample and 0.5 mL of a trimethylsilylating agent ("Silylating Agent TH" manufactured by Kanto Chemical Co., Inc.) were added into a glass sample bottle, followed by hermetic sealing, and the glass sample bottle was heated at 70°C for 15 minutes. 1.0 mL of water and 1.5 mL of hexane were added to the mixture, followed by shaking. After leaving it to stand, the upper layer was subjected to gas-liquid chromatography (GLC) to perform analysis.

(ii) Fatty acid composition

A fatty acid methyl ester was prepared in accordance with "Preparation method of fatty acid methyl ester (2.4.1.-1996)" in "Standard Methods for the Analysis of Fats, Oils and Related Materials" edited by Japan Oil Chemists' Society, and the obtained sample was measured by American Oil Chemists. Society Official Method Ce 1f-96 (GLC method).

(iii) Hydroxyl value

In conformity with "Hydroxyl value (pyridine-acetic anhydride method 2.3.6.2-1996)" in "Standard Methods for the Analysis of Fats, Oils and Related Materials" edited by Japan Oil Chemists' Society, about 5 g of an oil or fat sample were weighed in a round-bottom flask with a long neck. 5 ml of an acetylating reagent were added to the oil or fat sample, and a small funnel was put in the neck of the flask. The bottom portion of the flask was immersed in a heating bath up to a depth of about 1 cm, and was heated to a temperature of 95 to 100°C. One hour later, the flask was taken out from the heating bath and was then cooled. 1 ml of distilled water was added into the flask through the funnel, and the flask was heated again in the heating bath for 10 minutes. The flask was cooled again to normal temperature, and the liquid condensed on the inside surface of the funnel and on the inside surface of the neck of the flask was washed down into the flask with 5 ml of neutral ethanol. The resulting liquid was subjected to titration with a 0.5 mol/L potassium hydroxide-ethanol reference solution by using a phenolphthalein indicator. Note that a blank test was performed simultaneously with the main test, and a value calculated from the results of the titration on the basis of the following equation was defined as "hydroxyl value (mg-KOH/g)" (OHV).

$$\texttt{Hydroxyl value=(A-B)} \times 28.5 \times \texttt{F/C+acid value}$$

(A: Amount (ml) of a 0.5 mol/L potassium hydroxide-ethanol reference solution used in a blank test, B: Amount (ml) of a 0.5 mol/L potassium hydroxide-ethanol reference solution used in a main test, F: Factor of a 0.5 mol/L potassium hydroxide-ethanol reference solution, and C: Collection amount (g) of a sample)

(iv) Quantitative determination of bisbentiamine disulfide

0.3 g of Tween 80, 5 ml of an acetate buffer solution, and 1 ml of a 10% solution of thiourea were added to 0.5 g of an oil or fat sample to adjust the pH of the mixture to 4.5.

3 ml of a 2.5% solution of Taka-Diastase were further added thereto, and the whole was left to stand at 40°C overnight. 5 ml of 2N hydrochloric acid and 70 ml of ethanol were added thereto, and the resulting mixture was subjected to reaction treatment at 70°C for 30 minutes while sometimes stirring. The volume of the mixture was fixed at 100 ml with distilled water, followed by filtration, yielding an extraction liquid. 1 ml of a 0.7% solution of cysteine and 1 ml of a 4 N solution of sodium hydroxide were added to 5 ml of the extraction liquid, and the mixture was subjected to reaction at room temperature for 30 minutes. After that, 1 ml of 4 N hydrochloric acid was added thereto, and the volume of the mixture was fixed at 20 ml with distilled water, yielding a solution. Then, the solution was subjected to HPLC.

HPLC conditions:

Column: ODS-3 (4.6 ID×250 mm), manufactured by GL Sciences Inc.
Column temperature: 40°C
Mobile phase: (0.01 mol sodium dihydrogen phosphate+0.15 mol/L sodium perchlorate) mixed solution (having a pH of 2.2):methanol=95:5
Flow rate of mobile phase: 1 ml/min
Injection amount: 20 $\mu$l
Detection: fluorescence detector Ex=375 nm, Em=440 nm
Reaction solution: 0.05% potassium ferricyanide+15% sodium hydroxide
Flow rate of reaction solution: 0.4 ml/min

(v) Quantitative determination of nicotinamide

2 ml of methanol were added to 1 g of an oil or fat sample, and the mixture was stirred well with a vortex mixer. After phase separation, the methanol phase was collected. 2 ml of methanol were added to the remaining solution, followed by extraction in the same manner as described above. Three extraction operations with methanol were carried out in total, and the resulting methanol phases were combined, followed by drying at room temperature under a nitrogen gas flow. The resulting dried substance was dissolved in 1 ml of acetonitrile, followed by washing three times each with 1 ml of hexane. The resulting acetonitrile phase after the washing was dried at room temperature under a nitrogen gas flow. The resulting dried substance was again dissolved in acetonitrile to fix the volume of the resulting solution at a certain level, and the solution was subjected to HPLC.

HPLC conditions:

Column: ODS-3 (4.6 ID×250 mm), manufactured by GL Sciences Inc.
Column temperature: 40°C
Mobile phase: mixed solvent of 0.05 N sodium dihydrogen phosphate (A) and methanol (B)
Gradient: A/B=95/5 (0 min) → A/B=5/95 (50 min)
Flow rate of mobile phase: 0.5 ml/min
Injection amount: 20 $\mu$l
Detection: UV detector 260 nm

(vi) Quantitative determination of ascorbyl palmitate

0.5 g of ascorbic acid and 20 ml of 80% ethanol were added to 5 g of an oil or fat sample, and the mixture was stirred with a vortex mixer. The mixture was left to stand still, causing phase separation, and then the ethanol layer was collected. 10 ml of 80% ethanol were added to the remaining solution, followed by the same operation as described above, and the ethanol layer was collected. 10 ml of 80% ethanol were added again to the remaining solution, followed by the same operation as described above. The resulting ethanol layers were combined and the volume of the mixture was fixed at 50 ml with a 0.05 N acetate buffer solution (having a pH of 4) (buffer solution A), followed by filtration with a glass filter. 2 ml of the resulting filtrate were loaded into solid phase extraction column (Sep-Pak C18) prepared by preliminary treatment with 10 ml of methanol and 5 ml of a buffer solution (A), followed by washing with 10 ml of distilled water and 5 ml of a mixed solution of buffer solution A:ethanol=1:1 and elution with 4 ml of methanol, thereby yielding an eluate. 0.2 ml of a 1% aqueous solution of homocysteine was added to the eluate, followed by reaction treatment at 40°C for 15 minutes. After that, the volume of the resultant mixture was fixed at 10 ml with a 1% aqueous solution of ascorbic acid, yielding a solution. Then, the solution was subjected to HPLC.

HPLC conditions:

Column: ODS-3 (4.6 ID×250 mm), manufactured by GL Sciences Inc.
Column temperature: 40°C

Mobile phase: mixed solution of methanol and 0.05 M acetic acid buffer (pH 6.5) at a ratio of 85:15
Flow rate of mobile phase: 1 ml/min
Injection amount: 20 μl
Detection: UV detector 265 nm

(vii) Measurement of amount of water

Titrando type 851 (manufactured by Metrohm Japan Ltd.) was used to measure the amount of water in an oil or fat composition by the Karl Fischer coulometric titration method.

(Preparation of raw material oil or fat (1))

100 parts by mass of mixed fatty acids of soybean oil fatty acids:rapeseed oil fatty acids=7:3 (mass ratio) and 15 parts by mass of glycerin were mixed, and the resulting mixture was subjected to an esterification reaction with an enzyme, yielding a diacylglycerol-containing oil or fat. The resulting esterified mixture was subjected to distillation to remove fatty acids and monoacylglycerols, and the resultant was subj ected to acid treatment (a 10% aqueous solution of citric acid was addedat 2%), water washing, and deodorization treatment, yielding an oil or fat (1). Table 1 shows the glyceride composition and fatty acid composition thereof.

Rapeseed oil (manufactured by The Nisshin OilliO Group, Ltd.) was used as a raw material oil or fat (2), and a glycerin fatty acid monoester (O-95R manufactured by Kao Corporation) was used as a raw material oil or fat (3). Table 1 shows the glyceride compositions and fatty acid compositions of the raw material oils or fats (2) and (3).

[Table 1]

|  |  | Oil or fat (1) | Oil or fat (2) | Oil or fat (3) |
|---|---|---|---|---|
| Glyceride composition (%) | MAG | 0.5 | 0.0 | 95.0 |
|  | DAG | 87.0 | 2.0 | 5.0 |
|  | TAG | 12.5 | 98.0 | 0.0 |
| Fatty acid composition (%) | C16:0 | 3.1 | 4.2 | 5.0 |
|  | C18:0 | 1.1 | 2.1 | 7.3 |
|  | C18:1 | 38.7 | 62.5 | 83.8 |
|  | C18:2 | 48.0 | 20.2 | 3.3 |
|  | C18:3 | 9.0 | 11.0 | 0.6 |
| MAG: Monoacylglycerol<br>DAG: Diacylglycerol<br>TAG: Triacylglycerol | | | | |

Material oils or fats (1) to (3) were mixed at the mass ratios shown in Table 2, thereby preparing oils or fats A to G. Table 2 shows the glyceride compositions and hydroxyl values of the oils or fats A to G.

[Table 2]

|  |  | Oil or Fat A | Oil or Fat B | Oil or Fat C | Oil or Fat D | Oil or Fat E | Oil or Fat F | Oil or Fat G |
|---|---|---|---|---|---|---|---|---|
| Blend (%) | Oil or Fat (1) | 100.0 | 75.0 | 50.0 | 25.0 | - | 10.0 | 0.0 |
|  | Oil or Fat (2) | - | 25.0 | 50.0 | 75.0 | 97.0 | 90.0 | 100.0 |
|  | Oil or Fat (3) | - | - | - | - | 3.0 | - | - |

(continued)

|  | | Oil or Fat A | Oil or Fat B | Oil or Fat C | Oil or Fat D | Oil or Fat E | Oil or Fat F | Oil or Fat G |
|---|---|---|---|---|---|---|---|---|
| Glyceride composition (%) | MAG | 0.5 | 0.4 | 0.3 | 0.1 | 3.0 | 0.1 | 0.0 |
| | DAG | 87.0 | 65.8 | 44.5 | 23.3 | 1.9 | 10.5 | 2.0 |
| | TAG | 12.5 | 33.9 | 55.3 | 76.6 | 95.1 | 89.5 | 98.0 |
| Hydroxyl value (mg-KOH/g) | | 80.3 | 60.7 | 41.1 | 21.4 | 11.2 | 9.7 | 1.8 |
| MAG: Monoacylglycerol<br>DAG: Diacylglycerol<br>TAG: Triacylglycerol | | | | | | | | |

(Solubility test)

After an oil or fat composition was heated and stirred, it was left to stand at room temperature for 24 hours, followed by observation of its state. The solubility of bisbentiamine disulfide was evaluated on the basis of the criteria shown below.

b: Bisbentiamine disulfide is dissolved transparently.
d: An oil or fat composition is cloudy and bisbentiamine disulfide remains partially undissolved.

(Sensory evaluation)

Specialized panel members ate 1 to 2 g of an oil or fat composition, and evaluated its bitterness on the basis of the criteria shown below.

3: Bitter
2: Slightly bitter
1: Not bitter

Example 1

Bisbentiamine disulfide (manufactured by Mitsubishi Tanabe Pharma Corporation) was added to each of oils or fats A to G at each mass ratio shown in Tables 3 and 4, and each resulting mixture was heated and stirred in a hot-water bath at 80°C for 2 hours, yielding each of oil or fat compositions 1 to 24. Tables 3 and 4 show the analysis values thereof.

Next, each oil or fat composition was used to perform the solubility test and the sensory evaluation of a bitterness described above. Tables 3 and 4 show the results.

[Table 3]

| | | Inventive Product | | | | | | | | | | | | | |
|---|---|---|---|---|---|---|---|---|---|---|---|---|---|---|---|
| Oil or fat composition | | 1 | 2 | 3 | 4 | 5 | 6 | 7 | 8 | 9 | 10 | 11 | 12 | 13 | 14 |
| Oil or fat used | | A | A | B | B | C | C | C | D | D | E | E | F | F | F |
| BTDS (ppm) | | 3,000 | 2,000 | 2,000 | 1,000 | 1,000 | 300 | 200 | 300 | 200 | 200 | 100 | 200 | 100 | 50 |
| In terms of thiamine (ppm) | | 2,640 | 1,760 | 1,760 | 880 | 880 | 264 | 176 | 264 | 176 | 176 | 88 | 176 | 88 | 44 |
| Hydroxyl value (mg-KOH/g) | | 80.3 | 80.3 | 60.7 | 60.7 | 41.1 | 41.1 | 41.1 | 21.4 | 21.4 | 11.2 | 11.2 | 9.7 | 9.7 | 9.7 |
| Amount of water (ppm) | | 480 | 420 | 350 | 420 | 310 | 380 | 440 | 400 | 360 | 460 | 320 | 350 | 320 | 380 |
| Evaluation | Solubility | b | b | b | b | b | b | b | b | b | b | b | b | b | b |
| | Bitterness | 2 | 1 | 2 | 1 | 2 | 1 | 1 | 2 | 1 | 2 | 1 | 2 | 1 | 1 |
| BTDS: Bisbentiamine disulfide | | | | | | | | | | | | | | | |

[Table 4]

| | | Comparative Product | | | | | | | | | |
|---|---|---|---|---|---|---|---|---|---|---|---|
| Oil or fat composition | | 15 | 16 | 17 | 18 | 19 | 20 | 21 | 22 | 23 | 24 |
| Oil or fat used | | A | B | B | C | D | E | F | G | G | G |
| BTDS (ppm) | | 7,500 | 5,000 | 3,000 | 2,000 | 1,000 | 300 | 300 | 200 | 100 | 50 |
| In terms of thiamine (ppm) | | 6,600 | 4,400 | 2,640 | 1,760 | 880 | 264 | 264 | 176 | 88 | 44 |
| Hydroxyl value (mg-KOH/g) | | 80.3 | 60.7 | 60.7 | 41.1 | 21.4 | 11.2 | 9.7 | 1.8 | 1.8 | 1.8 |
| Amount of water (ppm) | | 380 | 300 | 340 | 400 | 360 | 320 | 350 | 320 | 350 | 320 |
| Evaluation | Solubility | d | d | b | d | d | d | d | d | b | b |
| | Bitterness | 3 | 3 | 3 | 3 | 3 | 3 | 3 | 3 | 3 | 3 |
| BTDS: Bisbentiamine disulfide | | | | | | | | | | | |

As seen from Tables 3 and 4, the oil or fat composition according to the present invention was able to contain a vitamin B1 derivative dissolved at a high concentration and had a less bitterness. In the oil or fat compositions 15 to 24, an undissolved vitamin B1 derivative was observed.

Further, the oil or fat compositions 2, 4, 6, 7, 9, 11, 13, and 14 were found to be preferred edible oils, because the bitterness derived from a vitamin B1 derivative was further suppressed.

Example 2

Bisbentiamine disulfide (manufactured by Mitsubishi Tanabe Pharma Corporation) and L-ascorbyl palmitate (manufactured by DSM Nutritional Products Ltd.) or nicotinamide (manufactured by Sigma-Aldrich Co. LLC.) were added to an oil or fat C or G at each mass ratio shown in Table 5, and each resulting mixture was heated and stirred in a hot-water bath at 80°C for 2 hours, yielding each of oil or fat compositions 25 to 30. Table 5 shows the analysis values thereof.

Next, each oil or fat composition was used to perform the solubility test and the sensory evaluation of a bitterness in the same manner as described above. Table 5 shows the results.

[Table 5]

| | | Inventive Product | | Comparative Product | | | |
|---|---|---|---|---|---|---|---|
| Oil or fat composition | | 25 | 26 | 27 | 28 | 29 | 30 |
| Oil or fat used | | C | C | G | G | G | G |
| BTDS (ppm) | | 300 | 300 | 300 | - | 300 | - |
| In terms of thiamine (ppm) | | 264 | 264 | 264 | - | 264 | - |
| Nicotinamide (ppm) | | 3,000 | - | 3,000 | 3,000 | - | - |
| Ascorbyl palmitate (ppm) | | - | 5,000 | - | - | 5,000 | 5,000 |
| In terms of ascorbic acid (ppm) | | - | 2,125 | - | - | 2,125 | 2,125 |
| Hydroxyl value (mg-KOH/g) | | 41.1 | 41.1 | 1.8 | 1.8 | 1.8 | 1.8 |
| Amount of water (ppm) | | 400 | 480 | 380 | 350 | 420 | 460 |
| Evaluation | Solubility | b | b | d | d | d | d |
| | Bitterness | 1 | 1 | 3 | 3 | 3 | - |
| BTDS: Bisbentiamine disulfide | | | | | | | |

As seen from Table 5, the oil or fat composition according to the present invention was able to contain a vitamin B1 derivative and ascorbyl palmitate or nicotinamide each dissolved at a high concentration and had a less bitterness.

Example 3

Nicotinamide (manufactured by Sigma-Aldrich Co. LLC.) was added to each of oils or fats A to G at each mass ratio shown in Tables 6 and 7, and each resulting mixture was heated and stirred in a hot-water bath at 80°C for 2 hours, yielding each of oil or fat compositions 31 to 52. Tables 6 and 7 show the analysis values thereof.

Next, each oil or fat composition was used to perform the solubility test and the sensory evaluation of a bitterness as described above. Tables 6 and 7 show the results.

[Table 6]

| | | Inventive Product | | | | | | | | | | | | | |
|---|---|---|---|---|---|---|---|---|---|---|---|---|---|---|---|
| Oil or fat composition | | 31 | 32 | 33 | 34 | 35 | 36 | 37 | 38 | 39 | 40 | 41 | 42 | 43 | 44 | 45 |
| Oil or fat used | | A | A | A | A | B | B | B | B | C | C | C | D | D | E | F |
| Nicotinamide (ppm) | | 15,00 0 | 10,00 0 | 7,500 | 5,000 | 7,500 | 5,000 | 4,000 | 3,000 | 4,000 | 3,000 | 2,000 | 1,500 | 1,000 | 1,000 | 1,000 |
| Hydroxyl value (mg-KOH/g) | | 80.3 | 80.3 | 80.3 | 80.3 | 60.7 | 60.7 | 60.7 | 60.7 | 41.1 | 41.1 | 41.1 | 21.4 | 21.4 | 11.2 | 9.7 |
| Amount of water (ppm) | | 420 | 450 | 450 | 460 | 440 | 430 | 380 | 390 | 420 | 360 | 360 | 420 | 320 | 340 | 320 |
| Evaluation | Solubility | b | b | b | b | b | b | b | b | b | b | b | b | b | b | b |
| | Bitterness | 3 | 3 | 3 | 1 | 3 | 2 | 1 | 1 | 2 | 1 | 1 | 1 | 1 | 1 | 1 |

EP 2 556 751 B1

[Table 7]

| | | Comparative Product | | | | | | |
|---|---|---|---|---|---|---|---|---|
| Oil or fat composition | | 46 | 47 | 48 | 49 | 50 | 51 | 52 |
| Oil or fat used | | A | B | C | D | E | F | G |
| Nicotinamide (ppm) | | 20,00 0 | 10,00 0 | 5,000 | 2,000 | 1,500 | 1,500 | 1,000 |
| Hydroxyl value (mg-KOH/g) | | 80.3 | 60.7 | 41.1 | 21.4 | 11.2 | 9.7 | 1.8 |
| Amount of water (ppm) | | 380 | 300 | 400 | 360 | 320 | 350 | 320 |
| Evaluation | Solubility | d | d | d | d | d | d | d |
| | Bitterness | 3 | 3 | 3 | 3 | 3 | 3 | 3 |

As seen from Table 6, the oil or fat composition according to the present invention was able to contain nicotinamide dissolved at a high concentration. In the oil or fat compositions 46 to 52, undissolved nicotinamide was observed.

Further, the oil or fat compositions 34 and 36 to 45, in particular, the oil or fat compositions 34, 37, 38, and 40 to 45 were found to be preferred edible oils, because the bitterness derived from nicotinamide was further suppressed.

Example 4

Nicotinamide (manufactured by Sigma-Aldrich Co. LLC.) and bisbentiamine disulfide (manufactured by Mitsubishi Tanabe Pharma Corporation, in terms of thiamine) were added to an oil or fat C or G at each mass ratio shown in Table 8, and each resulting mixture was heated and stirred in a hot-water bath at 80°C for 2 hours, yielding each of oil or fat compositions 53 to 56. Table 8 shows the analysis values thereof.

Next, each of the oil or fat compositions 53 to 56 were used to perform the solubility test and the sensory evaluation of a bitterness in the same manner as described above. Table 8 shows the results.

[Table 8]

| | | Inventive Product | Comparative Product | | |
|---|---|---|---|---|---|
| Oil or fat composition | | 53 | 54 | 55 | 56 |
| Oil or fat used | | C | G | G | G |
| Nicotinamide (ppm) | | 3,000 | 3,000 | - | 3,000 |
| In terms of thiamine (ppm) | | 300 | 300 | 300 | - |
| Hydroxyl value (mg-KOH/g) | | 41.1 | 1.8 | 1.8 | 1.8 |
| Amount of water (ppm) | | 400 | 380 | 350 | 420 |
| Evaluation | Solubility | b | d | d | d |
| | Bitterness | 1 | 3 | 3 | 3 |

As seen from Table 8, the oil or fat composition according to the present invention was able to contain nicotinamide and a vitamin B1 derivative each dissolved at a high concentration and had a less bitterness.

## Claims

1. An oil or fat composition, comprising a vitamin B1 derivative or a salt thereof at a content of 44 to 8,000 ppm in terms of thiamine and having a hydroxyl value of 9 to 100 mg-KOH/g,
   wherein the content C (ppm) of the vitamin B1 derivative or the salt thereof in terms of thiamine and the hydroxyl value X (mg-KOH/g) satisfy a relationship of the following expression (1) :

$$[Ln(C/143)]/X \leq 0.044 \quad (1),$$

where Ln represents a natural logarithm.

2. The oil or fat composition according to claim 1,
   wherein the content C (ppm) of the vitamin B1 derivative or the salt thereof in terms of thiamine and the hydroxyl value X (mg-KOH/g) satisfy a relationship of the following expression (2) :

$$[Ln(C/82)]/X \leq 0.04 \quad (2),$$

where Ln represents a natural logarithm.

3. The oil or fat composition according to claim 1 or 2, wherein the vitamin B1 derivative comprises bisbentiamine disulfide.

4. The oil or fat composition according to any one of claims 1 to 3, comprising diacylglycerols at 10 to 99 mass%.

5. The oil or fat composition according to any one of claims 1 to 4, further comprising vitamin C, a derivative thereof, or nicotinamide.

6. An oil or fat composition, comprising nicotinamide at a content of 1,000 to 20,000 ppm and having a hydroxyl value of wherein the content C (ppm) of nicotinamide and the hydroxyl value X (mg-KOH/g) satisfy a relationship of the following expression (3):

$$[Ln(C/850)]/X \leq 0.038 \quad (3),$$

where Ln represents a natural logarithm.

7. The oil or fat composition according to claim 6,
   wherein the content C (ppm) of nicotinamide and the hydroxyl value X (mg-KOH/g) satisfy a relationship of the following expression (4):

$$[Ln(C/679)]/X \leq 0.039 \quad (4),$$

where Ln represents a natural logarithm.

8. The oil or fat composition according to claim 7,
   wherein the content C (ppm) of nicotinamide and the hydroxyl value X (mg-KOH/g) satisfy a relationship of the following expression (5):

$$[Ln(C/854)]/X \leq 0.031 \quad (5),$$

where Ln represents a natural logarithm.

9. The oil or fat composition according to claim 7,
   wherein the content C (ppm) of nicotinamide and the hydroxyl value X (mg-KOH/g) satisfy a relationship of the following expression (6) :

$$[Ln(C/859)]/X \leq 0.024 \quad (6),$$

where Ln represents a natural logarithm.

10. The oil or fat composition according to claim 8 or 9, comprising nicotinamide at 1,000 to 4,500 ppm.

11. The oil or fat composition according to any one of claims 6 to 10, comprising diacylglycerols at 10 to 99 mass%.

12. The oil or fat composition according to any one of claims 6 to 11, further comprising a vitamin B1 derivative or a salt thereof.

13. The oil or fat composition according to claim 12, wherein the vitamin B1 derivative comprises bisbentiamine disulfide.

14. The oil or fat composition according to any one of claims 1 to 13, which is an edible oil.

15. The oil or fat composition according to any one of claims 1 to 14, wherein an amount of water in the oil or fat composition is 15,000 ppm or less.

16. The oil or fat composition according to any one of claims 1 to 14, wherein an amount of water in the oil or fat composition is 10,000 ppm or less.

17. The oil or fat composition according to any one of claims 1 to 14, wherein an amount of water in the oil or fat composition is 3,000 ppm or less.

18. A method for suppressing a bitterness of a vitamin B1 derivative, a salt thereof, or nicotinamide, which comprises adding the vitamin B1 derivative, the salt thereof, or nicotinamide to an oil or fat composition having a hydroxyl value of 9 to 100 mg-KOH/g.

19. The method for suppressing a bitterness according to claim 18, wherein the oil or fat composition comprises diacylglycerols at 10 to 99 mass%.

20. Use of an oil or fat composition having a hydroxyl value of 9 to 100 mg-KOH/g for suppressing a bitterness of a vitamin B1 derivative, a salt thereof, or nicotinamide.

**Patentansprüche**

1. Öl- oder Fettzusammensetzung, die ein Vitamin B1-Derivat oder ein Salz davon mit einem Gehalt von 44 bis 8.000 ppm in Bezug auf Thiamin umfasst und einen Hydroxylwert von 9 bis 100 mg-KOH/g aufweist, worin der Gehalt C (ppm) des Vitamin B1-Derivats oder des Salzes davon in Bezug auf Thiamin und der Hydroxylwert X (mg-KOH/g) die Beziehung des folgenden Ausdrucks (1) erfüllen:

$$[Ln(C/143)]/X \leq 0{,}044 \qquad (1)$$

worin Ln den natürlichen Logarithmus darstellt.

2. Öl- oder Fettzusammensetzung gemäß Anspruch 1, worin der Gehalt C (ppm) des Vitamin B1-Derivats oder des Salzes davon in Bezug auf Thiamin und der Hydroxylwert X (mg-KOH/g) die Beziehung des folgenden Ausdrucks (1) erfüllen:

$$[Ln(C/82)]/X \leq 0{,}04 \qquad (2)$$

worin Ln den natürlichen Logarithmus darstellt.

3. Öl- oder Fettzusammensetzung gemäß Anspruch 1 oder 2, worin das Vitamin B1-Derivat Bisbentiamindisulfid umfasst.

4. Öl- oder Fettzusammensetzung gemäß irgendeinem der Ansprüche 1 bis 3, die Diacylglyceride zu 10 bis 99 Masse% umfasst.

**5.** Öl- oder Fettzusammensetzung gemäß irgendeinem der Ansprüche 1 bis 4, die weiterhin Vitamin C, ein Derivat davon oder Nikotinamid umfasst.

**6.** Öl- oder Fettzusammensetzung, die Nikotinamid in einem Gehalt von 1.000 bis 20.000 ppm umfasst und einen Hydroxylwert von 9 bis 100 mg-KOH/g aufweist,
worin der Gehalt C (ppm) an Nikotinamid und der Hydroxylwert X (mg-KOH/g) die Beziehung des folgenden Ausdrucks (3) erfüllen:

$$[Ln(C/850)]/X \leq 0,038 \qquad (3)$$

worin Ln den natürlichen Logarithmus darstellt.

**7.** Öl- oder Fettzusammensetzung gemäß Anspruch 6,
worin der Gehalt C (ppm) an Nikotinamid und der Hydroxylwert X (mg-KOH/g) die Beziehung des folgenden Ausdrucks (4) erfüllen:

$$[Ln(C/679)]/X \leq 0,039 \qquad (4)$$

worin Ln den natürlichen Logarithmus darstellt.

**8.** Öl- oder Fettzusammensetzung gemäß Anspruch 7,
worin der Gehalt C (ppm) an Nikotinamid und der Hydroxylwert X (mg-KOH/g) die Beziehung des folgenden Ausdrucks (5) erfüllen:

$$[Ln(C/854)]/X \leq 0,031 \qquad (5)$$

worin Ln den natürlichen Logarithmus darstellt.

**9.** Öl- oder Fettzusammensetzung gemäß Anspruch 7,
worin der Gehalt C (ppm) an Nikotinamid und der Hydroxylwert X (mg-KOH/g) die Beziehung des folgenden Ausdrucks (6) erfüllen:

$$[Ln(C/859)]/X \leq 0,024 \qquad (6)$$

worin Ln den natürlichen Logarithmus darstellt.

**10.** Öl- oder Fettzusammensetzung gemäß Anspruch 8 oder 9, die Nikotinamid zu 1.000 bis 4.500 ppm umfasst.

**11.** Öl- oder Fettzusammensetzung gemäß irgendeinem der Ansprüche 6 bis 10, die Diacylglyceride zu 10 bis 99 Masse% umfasst.

**12.** Öl- oder Fettzusammensetzung gemäß irgendeinem der Ansprüche 6 bis 11, die weiterhin ein Vitamin B1-Derivat oder ein Salz davon umfasst.

**13.** Öl- oder Fettzusammensetzung gemäß Anspruch 12, worin das Vitamin B1-Derivat Bisbentiamindisulfid umfasst.

**14.** Öl- oder Fettzusammensetzung gemäß irgendeinem der Ansprüche 1 bis 13, bei der es sich um ein Speiseöl handelt.

**15.** Öl- oder Fettzusammensetzung gemäß irgendeinem der Ansprüche 1 bis 14, worin die Menge an Wasser in der Öl- oder Fettzusammensetzung 15.000 ppm oder weniger beträgt.

**16.** Öl- oder Fettzusammensetzung gemäß irgendeinem der Ansprüche 1 bis 14, worin die Menge an Wasser in der Öl- oder Fettzusammensetzung 10.000 ppm oder weniger beträgt.

**17.** Öl- oder Fettzusammensetzung gemäß irgendeinem der Ansprüche 1 bis 14, worin die Menge an Wasser in der Öl- oder Fettzusammensetzung 3.000 ppm oder weniger beträgt.

**18.** Verfahren zur Unterdrückung der Bitterkeit eines Vitamin B1-Derivats, eines Salzes davon oder von Nikotinamid, das die Zugabe des Vitamin B1-Derivats, des Salzes davon oder von Nikotinamid zu einer Öl- oder Fettzusammensetzung mit einem Hydroxylwert von 9 bis 100 mg-KOH/g umfasst.

**19.** Verfahren zur Unterdrückung der Bitterkeit gemäß Anspruch 18, worin die Öl- oder Fettzusammensetzung Diacylglyceride zu 10 bis 99 Masse% umfasst.

**20.** Verwendung einer Öl- oder Fettzusammensetzung mit einem Hydroxylwert von 9 bis 100 mg-KOH/g zur Unterdrückung der Bitterkeit eines Vitamin B1-Derivats, eines Salzes davon oder von Nikotinamid.

**Revendications**

**1.** Composition d'huile ou de matière grasse, comprenant un dérivé de vitamine B1 ou un sel de celui-ci à une teneur de 44 à 8 000 ppm en termes de thiamine et ayant un indice d'hydroxyle de 9 à 100 mg de KOH/g, dans laquelle la teneur C (ppm) du dérivé de vitamine B1 ou du sel de celui-ci en termes de thiamine et l'indice d'hydroxyle X (mg de KOH/g) satisfont une relation de l'expression (1) suivante :

$$[Ln(C/143)]/X \leq 0,044 \qquad (1),$$

où Ln représente un logarithme naturel.

**2.** Composition d'huile ou de matière grasse selon la revendication 1, dans laquelle la teneur C (ppm) du dérivé de vitamine B1 ou du sel de celui-ci en termes de thiamine et l'indice d'hydroxyle X (mg de KOH/g) satisfont une relation de l'expression (2) suivante :

$$[Ln(C/82]/X \leq 0,04 \qquad (2),$$

où Ln représente un logarithme naturel.

**3.** Composition d'huile ou de matière grasse selon la revendication 1 ou 2, dans laquelle le dérivé de vitamine B1 comprend du disulfure de bisbentiamine.

**4.** Composition d'huile ou de matière grasse selon l'une quelconque des revendications 1 à 3, comprenant des diacylglycérols à 10 à 99 % en masse.

**5.** Composition d'huile ou de matière grasse selon l'une quelconque des revendications 1 à 4, comprenant en outre de la vitamine C, un dérivé de celle-ci, ou du nicotinamide.

**6.** Composition d'huile ou de matière grasse, comprenant du nicotinamide à une teneur de 1 000 à 20 000 ppm et ayant un indice d'hydroxyle de 9 à 100 mg de KOH/g, dans laquelle la teneur C (ppm) de nicotinamide et l'indice d'hydroxyle X (mg de KOH/g) satisfont une relation de l'expression (3) suivante :

$$[Ln(C/850)]/X \leq 0,038 \qquad (3),$$

où Ln représente un logarithme naturel.

7.  Composition d'huile ou de matière grasse selon la revendication 6, dans laquelle la teneur C (ppm) de nicotinamide et l'indice d'hydroxyle X (mg de KOH/g} satisfont une relation de l'expression (4) suivante :

$$[Ln(C/679)]/X \leq 0,039 \qquad (4),$$

où Ln représente un logarithme naturel.

8.  Composition d'huile ou de matière grasse selon la revendication 7, dans laquelle la teneur C (ppm) de nicotinamide et l'indice d'hydroxyle X (mg de KOH/g) satisfont une relation de l'expression (5) suivante :

$$[Ln(C/854)]/X \leq 0,031 \qquad (5),$$

où Ln représente un logarithme naturel.

9.  Composition d'huile ou de matière grasse selon la revendication 7, dans laquelle la teneur C (ppm) de nicotinamide et l'indice d'hydroxyle X (mg de KOH/g) satisfont une relation de l'expression (6) suivante :

$$[Ln(C/859)]/X \leq 0,024 \qquad (6),$$

où Ln représente un logarithme naturel.

10. Composition d'huile ou de matière grasse selon la revendication 8 ou 9, comprenant du nicotinamide à 1 000 à 4 500 ppm.

11. Composition d'huile ou de matière grasse selon l'une quelconque des revendications 6 à 10, comprenant des diacylglycérols à 10 à 99 % en masse.

12. Composition d'huile ou de matière grasse selon l'une quelconque des revendications 6 à 11, comprenant en outre un dérivé de vitamine B1 ou un sel de celui-ci.

13. Composition d'huile ou de matière grasse selon la revendication 12, dans laquelle le dérivé de vitamine B1 comprend le disulfure de bisbentiamine.

14. Composition d'huile ou de matière grasse selon l'une quelconque des revendications 1 à 13, qui est une huile comestible.

15. Composition d'huile ou de matière grasse selon l'une quelconque des revendications 1 à 14, dans laquelle une quantité d'eau dans la composition d'huile ou de matière grasse est de 15 000 ppm ou moins.

16. Composition d'huile ou de matière grasse selon l'une quelconque des revendications 1 à 14, dans laquelle une quantité d'eau dans la composition d'huile ou de matière grasse est de 10 000 ppm ou moins.

17. Composition d'huile ou de matière grasse selon l'une quelconque des revendications 1 à 14, dans laquelle une quantité d'eau dans la composition d'huile ou de matière grasse est de 3000 ppm ou moins.

18. Procédé pour supprimer une amertume d'un dérivé de vitamine B1, d'un sel de celui-ci, ou du nicotinamide, qui comprend l'ajout du dérivé de vitamine B1, du sel de celui-ci, ou de nicotinamide à une composition d'huile ou de matière grasse ayant un indice d'hydroxyle de 9 à 100 mg de KOH/g.

19. Procédé pour supprimer une amertume selon la revendication 18, dans laquelle la composition d'huile ou de matière grasse comprend des diacylglycérols à 10 à 99 % en masse.

**20.** Utilisation d'une composition d'huile ou de matière grasse ayant un indice d'hydroxyle de 9 à 100 mg de KOH/g pour supprimer une amertume d'un dérivé de vitamine B1, d'un sel de celui-ci, ou du nicotinamide.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 8173035 A

**Non-patent literature cited in the description**

- Standard Methods for the Analysis of Fats, Oils and Related Materials. 2003
- Preparation method of fatty acid methyl ester (2.4.1.-1996). Standard Methods for the Analysis of Fats, Oils and Related Materials

- Hydroxyl value (pyridine-acetic anhydride method 2.3.6.2-1996). Standard Methods for the Analysis of Fats, Oils and Related Materials